# EUROPEAN PATENT APPLICATION

(11) **EP 4 344 675 A1**
(43) Date of publication of application: **03.04.2024**
(21) Application number: 22857722.7
(22) Date of filing: 15.08.2022
(51) Int. Cl.: A61F 2/06, A61F 2/07, A61F 2/966

(54) **MEDICAL DEVICE**

(30) Priority: 17.08.2021 CN 202110940080
(71) Applicant: Shanghai MicroPort Endovascular MedTech (Group) Co., Ltd., Shanghai 201318 (CN)
(72) Inventor: WU, Weiyi, Shanghai 201318 (CN); ZHU, Yongfeng, Shanghai 201318 (CN); ZHAO, Mingjie, Shanghai 201318 (CN); ZHANG, Bowei, Shanghai 201318 (CN); ZHU, Qing, Shanghai 201318 (CN)
(74) Representative: Patentship Patentanwaltsgesellschaft mbH
(86) International application number: PCT/CN2022/112357
(87) International publication number: WO 2023/020410

(57) **Abstract**

A medical device (100), comprising: a stent graft (110), defined thereon with an opening (111); a catheter (120), configured to be partially arranged inside the stent graft (110); and a restraint member (130), configured to apply a radial compression force to the stent graft (110), so as to radially compress the stent graft (110) into a compressed configuration; the medical device (100) is configured so that the opening (111) is in postional correspondence with a distal end of the catheter (120) when the stent graft (110) is in the compressed configuration; the distal end refers to an end that first enters a body of a patient during use of the medical device (100). The medical device (100) can be used for the treatment for vascular lesions involving the aortic arch, which is favorable for establishing access in the branch vessel and shortening the operation duration.

## Description

### TECHNICAL FIELD

The present invention relates to the technical field of medical instruments and, more specifically, to a medical device.

### BACKGROUND

Arterial vascular lesions involving the aortic arch are very complex and dangerous, and doctors often choose between thoracotomy and minimally invasive interventional procedures. Compared with thoracotomy, minimally invasive interventional procedures have the advantages of less trauma and faster postoperative recovery, but it also has some problems, especially on the treatment for branch vessels, which has always been the design focus of various products.

When doctors perform minimally invasive interventional procedures, most of them use DIY straight-tube stent grafts or aortic stent graft systems integrated with branch stents. However, no matter which stent system is used, the establishment of access in branch vessel is very important. In the prior art, during the establishment of the access in the branch vessel, the operation of introducing the branch vessel is generally carried out by means of a guidewire and a catheter, for example, from the lower extremity artery (e.g., the femoral artery) to the branch artery through the aorta, or from the upper extremity arteries and carotid arteries down into the aorta through the branch vessels. The specific operation generally includes implanting a main stent graft with an opening thereon, selecting a catheter that adapts to the angle of the branch vessel, guiding the catheter to the vicinity of the branch vessel, and aligning the angle of the distal end of the catheter to the branch vessel, introducing the guidewire into the branch vessel from the catheter to complete the establishment of the access in the branch vessel, and then implanting the branch stent into the corresponding branch vessel along the guidewire.

In the process of establishing the access in the branch vessel as mentioned above, the opening of the main stent graft needs to be aligned with the branch vessel, otherwise the opening of the branch vessel would be covered by the main section of the stent graft, which makes it more difficult to introduce the catheter and the guidewire into the branch vessel, prolonging the operation duration.

### SUMMARY OF THE INVENTION

An object of the present invention is to provide a medical device, which can be used for minimally invasive interventional therapy for lesions involving aortic arch, can effectively reduce the difficulty in introducing a guidewire into branch vessels, facilitate the establishment of the access in branch vessel, and shorten operation duration.

In order to achieve the above object, the present invention provides a medical device, comprising: a stent graft, defined thereon with an opening; a catheter, configured to be partially arranged inside the stent graft; and a restraint member, configured to apply a radial compression force to the stent graft, so as to radially compress the stent graft into a compressed configuration; wherein the medical device is configured so that the opening is in postional correspondence with a distal end of the catheter when the stent graft is in the compressed configuration; the distal end refers to an end that first enters a body of a patient during use.

Optionally, the stent graft is further configured to apply pressure to the catheter so that the catheter is kept stationary relative to the stent graft.

Optionally, the distal end of the catheter passes through the opening and protrudes out of the stent graft.

Optionally, the catheter comprises a main section and a distal section connected to a distal end of the main section at an angle, the main section partially arranged inside the stent graft, and the distal section passing through the opening and protruding out of the stent graft.

Optionally, the main section has an axis parallel to an axis of the stent graft.

Optionally, an obtuse angle ranging from 150° to 180° is formed between an axis of the main section and an axis of the distal section.

Optionally, the medical device further comprises a positioning member having a proximal end connected to the distal end of the catheter, and a distal end restrained on an outer surface of the stent graft by the restraint member.

Optionally, the positioning member and the catheter are separately formed and connected together; or, the positioning member and the catheter are integrally formed.

Optionally, a visualization element is provided on an outer surface of the distal end of the catheter.

Optionally, the medical device further comprises an inner liner configured to be inserted in the catheter.

Optionally, the medical device further comprises a delivery device that comprises an inner tube, the stent graft sleeved over the inner tube, and the catheter arranged between the inner tube and the stent graft.

Optionally, the catheter is connected to the inner tube.

Compared with the prior art, the medical device of the present invention has the following advantages: the aforementioned medical device includes a stent graft, a catheter and a restraint member; the stent graft is defined thereon with an opening; the catheter is used to partially arranged inside the stent graft; the restraint member is used to apply a radial compression force to the stent graft, so that the stent graft is radially compressed into a compressed configuration; the medical device is configured so that the opening is in positional correspondence with the distal end of the catheter when the stent graft is in the compressed configuration, and the distal end refers to the end that first enters the patient's body during use. Further, the stent graft preferably applies pressure to the catheter, so that the catheter is kept stationary relative to the stent graft. In this way, the stent graft and the catheter can be delivered into the aorta synchronously, and the user can, before the stent graft is released, introduce a guidewire along the catheter into the aorta and let the guidewire pass through the opening on the stent graft so as to introduce it into the branch vessel, which simplifies the operation for introducing the catheter and guidewire into the branch vessel. It also avoids the difficulty of superselection caused by the release of the stent graft and the misalignment between the opening and the branch vessel, which is conducive to the establishment of access in the branch vessel and shortens the operation duration.

### BRIEF DESCRIPTION OF THE DRAWINGS

The present invention will be better understood with reference to the accompanying drawings provided without limiting the invention.
Fig. 1 is a schematic structural diagram of a medical device according to the first embodiment of the present invention;
Fig. 2 is a schematic structural diagram of a catheter of the medical device according to the first embodiment of the present invention;
Fig. 3 is a schematic diagram showing an application scenario of the medical device according to the first embodiment of the present invention;
Fig. 4 is a schematic diagram showing an application scenario of the medical device according to the first embodiment of the present invention, in which the guidewire has been introduced into the branch vessel.
Fig. 5 is a schematic structural diagram of a medical device according to the second embodiment of the present invention;
Fig. 6 is a schematic diagram showing a partial structure of the medical device according to the thrid embodiment of the present invention; and
Fig. 7 is a schematic diagram showing a partial structure of the medical device according to the thrid embodiment of the present invention, in which the perspective is different from that in Fig. 6.

List of Reference Numerals: 100: medical device, 110: stent graft, 111: opening, 120: catheter, 121: main section, 122: distal section, 123: inclined surface, 130: restraint member, 140: visualization element, 150: positioning member; 200: guidewire.

### DETAILED DESCRIPTION

The embodiments of the present invention are described below through specific examples, and those skilled in the art can easily understand other advantages and effects of the present invention from the disclosure in this specification. The present invention can also be implemented or applied through other different specific embodiments, and various details in this specification can also be modified or changed based on different viewpoints and applications without departing from the spirit of the present invention. It should be noted that the drawings provided in embodiments are only to illustrate the basic concept of the present invention in a schematic way, so the drawings only show the components related to the present invention rather than the number, shape and the number of components in actual implementation. The type, quantity and proportion of each component can be changed at will in actual implementation, and the layout of components may also be more complicated.

In addition, each embodiment of the following description has one or more technical features, but this does not mean that the person using the present invention must implement all the technical features in any embodiment at the same time, or can only implement a part or all the technical features in different embodiments separately. In other words, under the premise of possible implementation, those skilled in the art can selectively implement some or all of the technical features in any embodiment, or the combination of some or all of the technical features in the multiple embodiments is selectively implemented, according to the disclosure of the present invention and depending on design specifications or implementation requirements, thereby increasing the flexibility of the implementation of the present invention.

As used in this specification, the singular forms "a", "an", and "the" include plural referents, and the plural form "a plurality" includes two or more referents unless the specification clearly dictates otherwise. As used in this specification, the term "or" is generally employed in its sense including "and/or" unless the specification clearly dictates otherwise, and the terms "installed", "coupled", "connected" shall be understood in a broad sense, for example, it may be a fixed connection, a detachable connection, or an integral connection. It can be a mechanical connection or an electrical connection. It can be directly connected, or indirectly connected through an intermediate medium, and it can be the internal communication between two elements or the interaction between the two elements. For those of ordinary skill in the art, the specific meanings of the above terms in the present invention can be understood according to specific situations.

As used herein, a "proximal end" and a "distal end" are relative orientations, relative positions and directions of elements or actions relative to each other from the perspective of a doctor using the medical instruments. Although the "proximal end" and the "distal end" are not restrictive, the "proximal end" generally refers to the end of the medical device that is close to the doctor during normal operation, while the "distal end" generally refers to the end that first enters the patient. The patients here can be humans or other animals.

The core idea of the present invention is to provide a medical device, which includes a stent graft, a catheter, and a restraint member. The stent graft is defined thereon with an opening, and the catheter is configured to be partially arranged inside the stent graft. The restraint member is configured to apply a radial compression force to the stent graft, so as to radially compress the stent graft into a compressed configration. The opening is in positional correspondence with the distal end of the catheter when the medical device is in the compressed configuration. The medical device can be used for the treatment for lesions involving the aortic arch. The catheter can be delivered together with the stent graft to the aortic arch. When the opening on the stent graft is located at the vicinity of the a branch vessel before the stent graft is released, the user introduces a guidewire to the aortic arch through the catheter, and then the guidewire can be introduced into the branch vessel. This can avoide the problem in the piror art that the opening of the branch vessel is partially covered due to the misalignment between the opening of the stent graft and the branch vessel during the inroduction of the catheter and the guidewire into the branch vessel after the stent graft is released. Preferably, the stent graft also applies pressure to the catheter so that the catheter is kept stationary relative to the stent graft. This avoids the misalignment between the distal end of the catheter and the opening, which may result from displacement between the stent graft and the catheter.

To make the objectives, advantages and features of the present invention clearer, the present invention will be further described in detail below with reference to the figures. It is noted that the figures are provided in a very simplified form not necessarily presented to scale, with their only intention to facilitate convenience and clarity in explaining the disclosed embodiments. Throughout the figures, like reference numerals indicate the same or analogous components or elements.

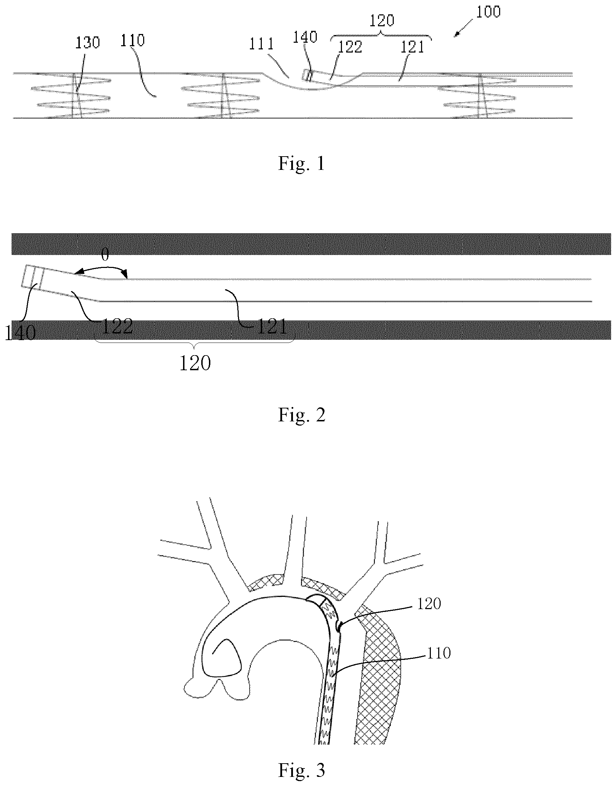

Fig. 1 is a schematic structural diagram of a medical device according to the first embodiment of the present invention. Referring to Fig. 1, the medical device 100 includes a stent graft 110, a catheter 120 and a restraint member 130. The stent graft 110 is defined thereon with a opening 111. The catheter 120 is configured to be partially arranged inside the stent graft 110. The restraint member 130 is configured for applying a radial compression force to the stent graft 110, so as to radially compress the stent graft 110 into a compressed configuration. The medical device 100 is configured such that the opening 111 is in positional correspondence with the distal end of the catheter 120 when the stent graft 110 is in the compressed configuration. Those skilled in the art should know that the catheter 120 has an inner cavity extending through the catheter along the axial direction thereof, and the distal end of the catheter 120 is the distal opening of the inner cavity. Therefore, the distal end of the catheter 120 here is in positional correspondence with the opening 111, which means that the distal end of the catheter 120 is in postional correspondence with the opening 111 in both the axial and circumferential directions of the stent graft 110, so that the stent graft 110 does not cover the distal opening of the inner cavity of the catheter 120. The distal end of the catheter 120 refers to the end of the catheter 120 that first enters the patient's body. The stent graft 110 may be a self-expanding stent, and the material of the catheter 120 may be a polymer material such as PA12 or Pebax7233.

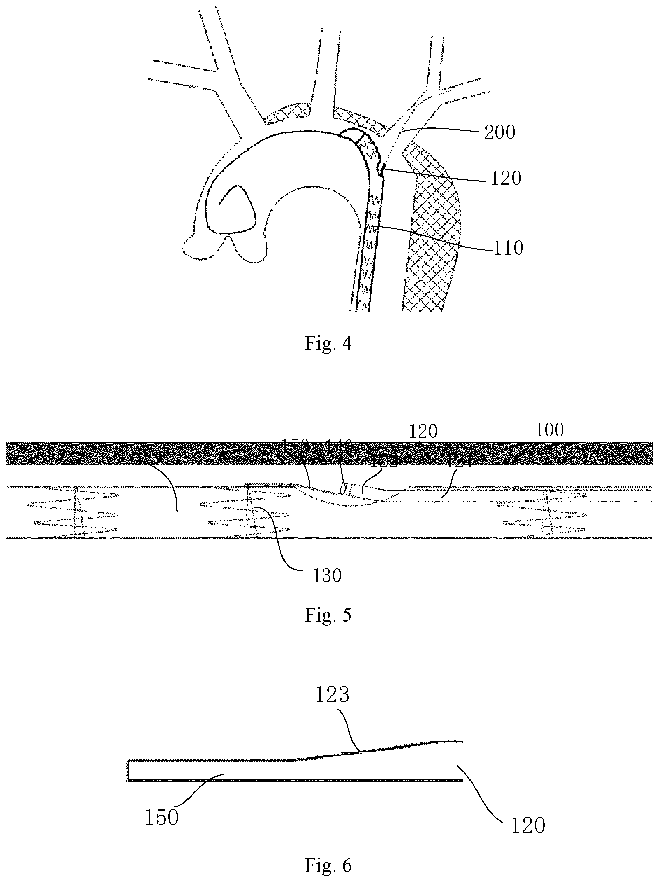

The medical device 100 can be used for treatments for lesions involving the aortic arch. During actual operation, the stent graft 110 is in a compressed configuration under the action of the restraint member 130, and is loaded together with the catheter 120 into a delivery device (not shown in the figures). The delivery device is configured to deliver the stent graft 110 together with the catheter 120 to the aortic arch of the patient, so that the opening 111 is located near a branch vessel. As shown in Figs. 3 and 4, since the distal end of the catheter 120 is arranged in positional correspondence with the opening 111, the user can, before the stent graft 110 is released, introduce a guidewire 200 through the inner cavity of the catheter 120 into the aortic arch and let the guidewire 200 protrude out from the distal end of the catheter 120 to enter the branch vessel. This is advantaged in that the stent graft 110 and the catheter 120 are delivered into the body at one time, avoiding secondary delivery, and also avoiding the occurrence of misalignment of the stent graft 110 with the branch vessel after release, resulting in difficulty in introducing the catheter 120 into the branch vessel, thereby avoiding the problem of difficulty in introducing the guidewire 200 into the branch vessel, which is beneficial to establish the access in the branch vessel and shorten the operation duration. Preferably, the stent graft 110 applies pressure to the catheter 120, so that the catheter 20 is kept stationary relative to the stent graft 110. This avoids a change of the position of the stent graft 110 relative to catheter 120, which may cause the misalignment between the distal end of the catheter 120 and the opening 111.

Optionally, the medical device 100 further includes the delivery device that includes an inner tube and an outer tube. The stent graft 110 is sleeved over the inner tube, and the outer tube is sleeved over the inner tube and is further configured to cover the stent graft 110.

Preferably, referring back to Fig. 1 in conjunction with Fig. 2, the outer surface of the distal end of the catheter 120 is provided with a visualization element 140, which makes it convenient for the user to determine the positions of the distal end of the catheter 120 and the opening 111.

Preferably, please continue to refer to Figs. 1 and 2, the distal end of the catheter 120 passes through the opening 111 and protrudes out of the stent graft 110. Specifically, the catheter 120 may include a main section 121 and a distal section 122. Preferably, the distal section 122 is connected with the distal end of the main section 121 at an angle. The main section 121 is partially arranged in the stent graft 110, and the distal section 122 passes through the opening 111 and protrudes out of the stent graft 110. That is, the end of the distal section 122 away from the main section 121 is the distal end of the catheter 120. In this way, it is more convenient for the guidewire 200 to be introduced into the branch vessel. In this embodiment, an obtuse angle θ is formed between the axis of the main section 121 and the axis of the distal section 122, and the obtuse angle θ is preferably 150°~180°, and the axis of the main section 121 may be parallel to the axis of the stent graft 110.

Next, a description is provided about the method for operating the medical device, which includes the steps as follows. Firstly, the stent graft 110 is sleeved over the inner tube of the delivery device, and the catheter 120 is partially inserted in the stent graft 110. Specifically, the proximal end of the main section 121 of the catheter 120 is located outside the stent graft 110 and extends to the proximal end of the delivery device, the distal end of the main section 121 is located between the stent graft 110 and the inner tube, and the distal section 122 of the catheter 120 protrudes out of the stent graft 110 from the opening 111. The restraint member 130 is then used to restrain the stent graft 110. In this embodiment, the restraint member 130 may be selected from any type of binding coil in the prior art, as long as it can radially compress and secure the stent graft 110 on the inner tube. That is, the restraint member 130 can apply a radial compression force to the stent graft 110 outside the stent graft 110, so as to compress and secure the stent graft 110 on the inner tube. Optionally, the stent graft 110 can also apply pressure to the catheter 120 under the action of the restraint member 130, so that the stent graft 110 and the catheter 120 are kept stationary with each other under the action of the pressure. Alternatively, in practice, the catheter 120 can be connected to the inner tube in other ways, for example, by binding or gluing, so that the catheter 120 is secured between the stent graft 110 and the inner tube, and thus the catheter 120 is kept stationary relative to the stent graft 110. Finally, the outer tube is covered over the stent graft 110.

Next, the stent graft 110 and the catheter 120 are delivered to the aortic arch of the patient in a conventional way, and the outer tube is withdrawn to expose the stent graft 110.

Next, the position of the stent graft 110 is adjusted so that the opening 111 is near a branch vessel, and the distal end of the catheter 120 is aligned with the branch vessel (as shown in Fig. 3).

Next, a guidewire 200 is delivered into the patient's body along the inner cavity of the catheter 120, and the guidewire 200 protrudes out from the distal end of the inner cavity and is directly introduced into the branch vessel (as shown in Fig. 4).

Then, the stent graft 110 is relased from the restraint member 130, and the stent graft 110 is deployed. So far, the access in the branch vessel is established, and the user can continue to introduce the branch stent into the branch vessel.

Fig. 5 is a schematic structural diagram of a medical device according to the second embodiment of the present invention. Please refer to Fig. 5, the difference between this embodiment and the first embodiment is that the medical device 100 further includes a positioning member 150. The proximal end of the positioning member 150 is connected with the distal end of the catheter 120, and the distal end of the positioning member 150 is restrained on the outer surface of the stent graft 110 by the restraint member 130. This ensures that the catheter 120 and the stent graft 110 remain stationary with each other, and prevents the catheter 120 from displacement during the delivery process, causing the distal end of the catheter 120 to deviate from the opening 111. It can be understood that the proximal end of the positioning member 150 should not be connected to the side of the catheter 120 away from the central axis of the stent graft 110, so as to avoid interference with the guidewire 200 and facilitate the introduction of the guidewire 200 into the brach vessel. Preferably, the proximal end of the positioning member 150 is connected to the side of the catheter 120 close to the central axis of the stent graft 110.

In this embodiment, the positioning member 150 may be formed separately from the catheter 120 and then connected together, and the positioning member 150 may be a metal wire or a polymer wire.

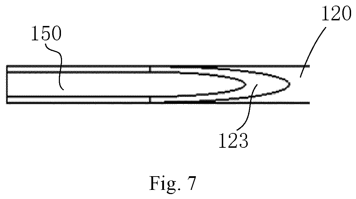

Figs. 6 and 7 are schematic diagrams showing a partial structure of the medical device according to the thrid embodiment of the present invention. As shown in Figs. 6 and 7, the difference between this embodiment and the second embodiment is that the positioning member 150 and the catheter 120 are integrally formed. Specifically, the positioning member 150 and the catheter 120 are formed from a single tube including a first part and a second part that are axially connected. The portion of the first part away from the central axis of the stent graft 110 is cut away, and the remaining portion of the first part close to the central axis of the stent graft 110 forms the positioning member 150 that can cover 1/4-1/2 of the circumference of the tube. The second part constitutes the catheter 120.

Further, a portion of the second part is also cut away, so that the distal end of the catheter 120 is formed with an inclined surface 123 connected to the positioning member 150 by a smooth transition. Therefore, the stress at the connection between the catheter 120 and the positioning member 150 is reduced, and the distal opening of the inner cavity of the catheter 120 can be enlarged, which makes it convenient to adjust the direction of the guidewire 200.

The difference between the medical device provided in the fourth embodiment of the present invention and the first embodiment is that the medical device 100 further includes an inner liner (not shown in the figures), and the inner liner is configured to be inserted in the inner cavity of the catheter 120. The inner liner is used in the process of assembling the medical device 100 and delivering the stent graft 110 into body of the patient. When the stent graft 110 is positioned, the inner liner is withdrawn. The inner liner is configured to prevent the catheter 120 from being compressed by the stent graft 110 to cause the close of the inner cavity, and from being bent and deformed when the catheter 120 is twisted in the stent graft 110.

The material of the inner liner can be a polymer such as Pebax7233, PTFE, PA12, etc. The outer diameter of the inner liner is smaller than or equal to the inner diameter of the catheter 120.

Although the present invention is disclosed above, it is not limited thereto. It is apparent that those skilled in the art can make various modifications and variations to the present invention without departing from the spirit and scope thereof. Accordingly, the invention is intended to embrace all such modifications and variations if they fall within the scope of the appended claims and equivalents thereof.

## Claims

1. A medical device, comprising:
a stent graft, defined thereon with an opening;
a catheter, configured to be partially arranged inside the stent graft; and
a restraint member, configured to apply a radial compression force to the stent graft, so as to radially compress the stent graft into a compressed configuration;
wherein the medical device is configured so that the opening is in postional correspondence with a distal end of the catheter when the stent graft is in the compressed configuration; the distal end refers to an end that first enters a body of a patient during use of the medical device.

2. The medical device of claim 1, wherein the stent graft is further configured to apply pressure to the catheter so that the catheter is kept stationary relative to the stent graft.

3. The medical device of claim 1, wherein the distal end of the catheter passes through the opening and protrudes out of the stent graft.

4. The medical device of claim 3, wherein the catheter comprises a main section and a distal section connected to a distal end of the main section at an angle, the main section partially arranged inside the stent graft, and the distal section passing through the opening and protruding out of the stent graft.

5. The medical device of claim 4, wherein the main section has an axis parallel to an axis of the stent graft.

6. The medical device of claim 4, wherein an obtuse angle ranging from 150° to 180° is formed between an axis of the main section and an axis of the distal section.

7. The medical device of any one of claims 1-6, further comprising a positioning member having a proximal end connected to the distal end of the catheter, and a distal end restrained on an outer surface of the stent graft by the restraint member.

8. The medical device of claim 7, wherein the positioning member and the catheter are separately formed and connected together; or, the positioning member and the catheter are integrally formed.

9. The medical device of any one of claims 1-6, wherein a visualization element is provided on an outer surface of the distal end of the catheter.

10. The medical device of any one of claims 1-6, further comprising an inner liner configured to be inserted in the catheter.

11. The medical device of any one of claims 1-6, further comprising a delivery device that comprises an inner tube, the stent graft sleeved over the inner tube, and the catheter arranged between the inner tube and the stent graft.

12. The medical device of claim 11, wherein the catheter is connected to the inner tube.
